# EUROPÄISCHE PATENTSCHRIFT

(11) **EP 2 092 334 B1**
(45) Veröffentlichungstag und Bekanntmachung des Hinweises auf die Patenterteilung: **08.02.2012**
(21) Anmeldenummer: 07847935.9
(22) Anmeldetag: 06.12.2007
(51) Int. Cl.: G01N 33/538, G01N 33/543

(54) **ANALYSEVERFAHREN UND PORTABLES ANALYSEGERÄT**
ANALYSIS METHOD, AND PORTABLE ANALYSIS DEVICE
METHODE D'ANALYSE ET DISPOSITIF D'ANALYSE PORTABLE

(30) Priorität: 08.12.2006 DE 102006058374
(43) Veröffentlichungstag der Anmeldung: 26.08.2009
(73) Patentinhaber: SAW Instruments GmbH, 53177 Bonn (DE)
(72) Erfinder: PERPEET, Markus, 42279 Wuppertal (DE); TEWES, Michael, 53332 Roisdorf (DE)
(74) Vertreter: Braun-Dullaeus, Karl-Ulrich
(86) Internationale Anmeldenummer: PCT/EP2007/063465
(87) Internationale Veröffentlichungsnummer: WO 2008/068315

(56) Entgegenhaltungen:
- EP-A- 0 811 847
- WO-A-95/32419

## Beschreibung

Die vorliegende Erfindung betrifft ein Verfahren zum Nachweis eines definierten biorelevanten Analyten, wobei der Analyt in eine mit spezifischen Bindungspartnern versehene Flüssigkeit eingebracht wird, wobei Moleküle des Analyten an Rezeptoren von Bindungspartnern selektiv koppeln, wobei die Flüssigkeit nach Eingabe des Analyten einer Filterstruktur ausgesetzt, insbesondere durch sie hindurch geleitet wird, in der Moleküle desselben Analyten fest gebunden sind, wobei die Filterstruktur Bindungspartner bindet, die freie Rezeptoren aufweisen, wobei Bindungspartner, welche die Filterstruktur durchdringen, nachfolgend mit einem Sensor nachgewiesen werden. Die Erfindung betrifft ebenso eine handhabbare Vorrichtung zur Durchführung des Verfahrens.

Es sind verschiedene Verfahren zum Nachweis biorelevanter Analyten bekannt. Ein Verfahren zum schnellen Nachweis solcher Analyte bedient sich speziell präparierter Papierstreifen, sogenannter Teststreifen, die in eine den Analyten enthaltende Flüssigkeit getaucht werden. Das Analyseergebnis ergibt sich aus dem Auftreten und dem Grad einer spezifischen Verfärbung des Teststreifens. Diese einfach zu handhabende Analyse lässt sich wegen der unterschiedlichen erhältlichen Teststreifen vielfältig eingesetzten. So werden Teststreifen für Messungen in Flüssigkeiten respektive wässrigen Lösung verwendet, um beispielsweise Drogen, verbotene Substanzen oder Krankheitsmarker in Speichel oder Urin nachzuweisen. Auf diese Art lassen sich auch Krankheitserreger im Trinkwasserwasser, Keime in Lebensmitteln oder Schadstoffe in Gewässern nachweisen.

Allerdings ergeben die Teststreifen nur in begrenztem Rahmen quantitative Ergebnisse. Diese werden meist über einen Vergleich der Farbe des Teststreifens mit einer Farbwerttabelle durchgeführt und sind entsprechend subjektiv, da der Vergleich signifikant von der Wahrnehmungsfähigkeit und der Einschätzung der den Test durchführenden Person abhängt. In einer besonders aufwendigen Ausführungsform wird das Testergebnis mit einem optischen Lesegerät ermittelt.

Aus der DE 196 22 503 A1 ist ein Verfahren zum Nachweis von Analyten bekannt, das sich mehrzoniger Chromatographie-Teststreifen bedient. Eine auf die erste Zone aufgebrachte Elutationsflüssigkeit wandert aufgrund kapilarer Kräfte zur benachbarten Zone, wo sie mit dem Analyten versetzt wird. In einer weiteren Zone des Teststreifens werden Analytmoleküle durch entsprechende Bindungspartner komplexiert, wobei die nicht komplexierten Bindungspartner in einer nachfolgenden Filterstruktur enthaltend immobilisierte Analytmoleküle festgehalten werden. Die Bindungspartner, die eine anschließende Zielzone erreichen, werden dort optisch nachgewiesen.

Aufgabe der Erfindung ist es nunmehr, ein solches Verfahren zu schaffen, das sich mit einfachen und einfach handhabbaren Mitteln durchführen lässt und das bei hoher Sensibilität schnell und zuverlässig zu Messergebnissen führt, die auch von quantitativer Aussagekraft sind. Zudem ist es die Aufgabe der Erfindung, ein handhabbares Gerät zur Umsetzung des Verfahrens vorzuschlagen.

Diese Aufgaben werden durch das Verfahren nach Anspruch 1 und durch das Gerät nach Anspruch 9 gelöst. Vorteilhafte Ausführungsformen der Erfindung sind in den jeweiligen Unteransprüchen genannt.

Ein wesentlicher Grundgedanke der Erfindung liegt in der Wahl respektive in der Ausbildung der Bindungspartner. Diese sind so auszubilden, dass sie sich durch ein von Außen angelegtes Feld beeinflussen lassen. Ein weiterer Gedanke liegt darin, dieses Feld so einzurichten, dass die Bindungspartner zum Sensor hin geführt und an oder auf einer sensitiven Oberfläche des Sensors konzentriert werden. Dadurch, dass die Bindungspartner aus der zur Verfügung stehenden Flüssigkeit heraus "gefischt" und an der Oberfläche des Sensors gebunden werden, kann die Sensibilität der Messung im Gegensatz zu bekannten Verfahren erheblich gesteigert werden. Die Bindung zwischen dem Bindungspartner und dem Molekül des Analyten ist vorteilhafterweise von immunologischer Natur.

Besonders einfach lässt die erfindungsgemäße Verfahrensweise bewerkstelligen, wenn als Bindungspartner spezielle Trägerpartikel eingesetzt werden, die mit auf den zu messenden Analyten sensiblen Rezeptoren versehen sind. Der Vorteil solcher Trägerpartikel liegt darin, dass diese im Hinblick auf die Wechselwirkung mit dem Feld ausgewählt werden können. Als von Außen anlegbare Felder kommen dabei vor allem solche in Betracht, die sich einfach handhaben und insbesondere ohne weiteres insbesondere für die Dauer der Messung zu- und abschalten lassen. Auch wenn das beispielsweise mit Zentrifugalbeschleunigung bewerkstelligt werden kann, ist es besonders vorteilhaft, wenn Magnetfelder und/oder elektrische Feldern eingesetzt werden. Ein genereller Vorteil der Nutzung solcher Felder liegt darin, dass nach Abschalten des Feldes die Oberfläche des Sensors gespült und damit der Sensor für die nächste Messung vorbereitet werden kann.

Im Hinblick darauf ist es besonders vorteilhaft, wenn Trägerpartikel aus einem magnetischem Material, insbesondere aus einem ferromagnetischem Material, verwendet werden, die durch Anlegen eines Magnetfeldes an die Oberfläche des Sensors geführt werden können. Solche Partikel mit Dimension vorteilhafterweise im Nano-Bereich sind kommerziell erhältlich. Es kann auch vorteilhaft sein, eine Suspension superparamagnetischer Nanoteilchen in einer Trägerflüssigkeit, eine sogenannte "magnetische Flüssigkeit" (Ferroflüid) zu verwenden.

Eine besonders vorteilhafte Ausführungsform des Systems basiert demnach auf dem Einsatz magnetischer Nanopartikel, deren Oberflächen mit biochemischen Rezeptoren ausgestattet sind. Über diese modifizierten Nanopartikel, werden die gesuchten Substanzen "aufgespürt" und messtechnisch zugänglich gemacht. Vorteilhafterweise wird als elektronischer Detektor ein Surface Acoustic Wave (SAW) basiertes massensensitives Sensorelement eingesetzt. Die Funktion solcher SAW Sensoren ist hinreichend bekannt.

Generell können die Nanopartikel einen magnetischen Kern, beispielsweise aus Magnetid (Fe₃O₄), aufweisen und von einer Primär-Partikelgröße zwischen 5 nm und 10 nm sein. Dabei sind jedoch auch Größen im Bereich von Mikrometern denkbar. Vorteilhafterweise haben die Trägerpartikel eine Größe von weniger als 1 Mikrometer insbesondere weniger als 100 Nanometer.

In einer vorteilhaften Ausführungsform sind die Partikel mit einer stabilisierenden Hüllschicht versehen, die beispielsweise aus einem biokompatiblen Polymer besteht. Über funktionelle Gruppen am Hüllpolymer können biologisch aktive Substanzen, insbesondere Proteine (z. B. Antikörper) und Nukleinsäuren (DNA, RNA) gezielt an die Partikel immobilisiert werden.

Die Trägerpartikel können auch aus einem Metall sein, wobei Technologien zur Immobilisierung von Rezeptormolekülen auf Metalloberflächen bekannt sind. Entsprechende "Assays", mit denen Rezeptormoleküle and die Partikeloberflächen gekoppelt werden können" werden kommerziell angeboten. Wenn Trägerpartikel aus elektrisch leitendem und/oder polarisierbarem Material verwendet werden, können sich auch durch Anlegen eines elektrischen Feldes auf der Oberfläche des Sensors gebunden werden.

Mit der Offenbarung ist ein komfortabel zu handhabendes Verfahren für die Detektion biorelevanter Substanzen, wie insbesondere von Drogen, sowie Lebensmittel- und Umweltgiften, geschaffen, das bei hoher Empfindlichkeit für den Einsatz in einem tragbaren Messgerät ("Handheld"), geeignet ist. Dabei liegt der Vorteil eines solchen Handhelds darin, dass es am "Point of Care" eingesetzt werden kann und dabei keiner weiteren Laboreinrichtungen bedarf. Damit stellt die Offenbarung eine Verbesserung der bekannten Teststreifen dar, wobei die Verbesserung insbesondere in der Möglichkeit einer hochsensitiven elektronischen Detektion und Quantifizierung der gesuchten Substanz(en) durch den Sensor liegt. Mit der Erfindung wird die Empfindlichkeit der bisherigen Verfahren erheblich gesteigert. Darüber hinaus wird die subjektive Komponente der Auswertung vermieden und das Messergebnis mit einer Zahl belegt.

Der mobile Einsatz ist möglich, da die Testergebnisse bereits nach kurzer Zeit vorliegen. Zudem ist das Verfahren insofern sicher, als die hochspezifischen Bindungspartner, insbesondere in Form von Antikörpern, einen zuverlässigen Nachweis einer Vielzahl von Analyten, insbesondere aller relevantren Drogenarten garantieren. Dabei lässt es die Offenbarung zu, dass in Kombinationstest mehre Analyte zeitgleich gemessen werden können, so dass Test auf mehrere Drogenarten möglich sind. Das Verfahren lässt sich außerdem kostengünstig umsetzen. Der Analyt, der sich in einer Körperflüssigkeit befindet, kann auf einfache Weise an der entsprechenden Körperregion eines Probanden abgenommen werden. Somit ersparen solche Schnelltests, wenn sie im Rahmen einer Rehabilitation eingesetzten werden, die aufwendigen klinische Tests. Bezüglich der Einsatzmöglichkeiten wird auch auf die DE 196 22 503 A1 Bezug genommen, in der einige Kombinationen von Analyt und Rezeptor offenbart sind.

Ein besonderer Vorteil der Offenbarung ist das universelle Messprinzip, das für verschiedenste Substanzen in den unterschiedlichsten Einsatzbereichen anwendbar ist. Dieser Vorteil kommt besonders zum tragen, wenn das handhabbare Gerät mit austauschbaren Messkartuschen ausgestattet werden kann, wobei die Spezifizierung über die die Trägerpartikel enthaltende Messkartusche geschieht. Diese enthält ein auf die gesuchte Substanz sensitives System. Die Messkartusche ist dabei vorteilhafterweise ein einmalig zu verwendendes Wegwerfprodukt. Damit ist mit dem Handheld ein universelles Analysesystem geschaffen das entsprechend der Wahl der Messkartusche für vielfältige Testaufgaben einsetzbar ist. Vorteilhafterweise sind die Nanopartikeldurchsetzte Pufferlösung sowie die Matrix zur Partikelfilterung sind in die einmalig zu verwendende Messkartusche integriert. Diese handhabbaren Analysegeräte sind für den Massenmarkt, außerhalb von Forschung und Entwicklung geeignet. Sie ersetzen damit die Teststreifen für schnelle Analysen vor Ort, als einmalig zu benutzende Wegwerfprodukte.

Basierend auf einer hochsensitiven elektronischen Detektion und Quantifizierung der gesuchten Substanz(en) bzw. deren Mengen wird das Analyseergebnis mit einer Zahl belegt. Darüber hinaus wird die Empfindlichkeit gegenüber bisherigen Teststreifenverfahren erheblich gesteigert. Das Messprinzip ist dabei so universell einsetzbar, dass es für verschiedenste Substanzen in den unterschiedlichsten Einsatzbereichen anwendbar ist. Die Spezifizierung erfolgt allein über eine spezielle Messkartusche, die das System auf die gesuchte Substanz sensitiviert. Damit steht vielfältigen Testaufgaben ein einzelnes, universelles Analysesystem gegenüber.

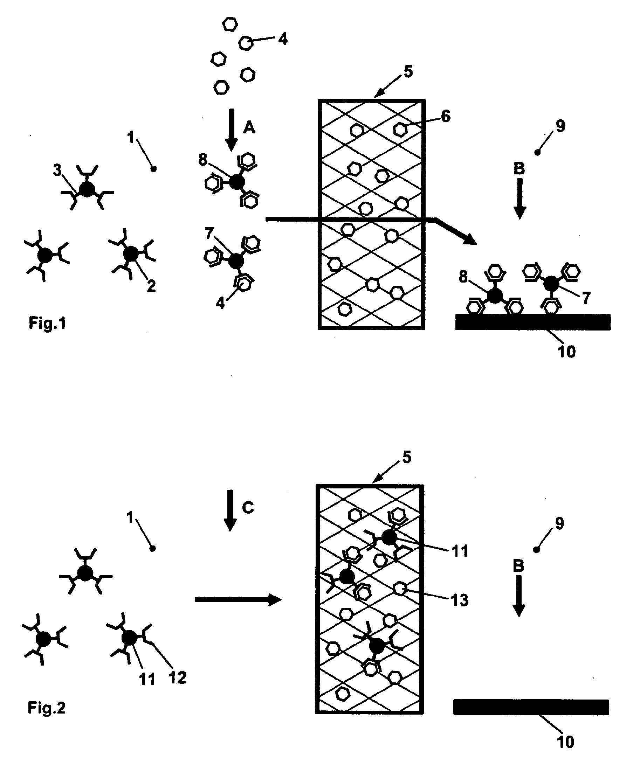

Nachfolgend wird die Erfindung anhand der Figuren 1 und 2 näher erklärt. Dabei zeigen
- **Figur 1:**: Verfahren bei vorhandenem Analyten und
- **Figur 2**:: Verfahren bei nicht vorhandenem Analyten.

In Figur 1 ist das Verfahren zum Nachweis eines definierten biorelevanten Analyten gezeigt, der in eine mit spezifischen Bindungspartner versehene Flüssigkeit 1, hier in eine wässrige Lösung, eingebracht wird, Die Flüssigkeit 1 befindet sich in einem nicht weiter dargestellten Behälter. In der Flüssigkeit 1 sind als Bindungspartner magnetische Nanopartikel als Trägerpartikel 2 vorhanden, deren Oberflächen mit auf den Analyten sensiblen Rezeptoren 3 versehen sind, wobei die Rezeptoren 3 von Antikörpern gebildet sind, die spezifisch an die gesuchte Substanz, den Analyt, binden. Wird nun eine Probe enthaltend Moleküle 4 des Analyten von Außen über einen nicht dargestellten Zugang in den mit Flüssigkeit 1 gefüllten Behälter eingebracht (Pfeil A), binden diese Moleküle 4 an die Rezeptoren 3 der Trägerpartikel 2 selektiv.

Nach Eingabe des Analyten wird die Flüssigkeit 1 durch eine Filterstruktur 5 geleitet, in deren Matrix Moleküle 6 desselben Analyten fest eingebunden sind. Da die Rezeptoren 3 der beiden in der Figur schematisch dargestellten Trägerpartikel 7 und 8 jedoch schon Moleküle 4 gebunden haben und somit "gesättigt" sind, wandern sie durch die Filterstruktur 5 hindurch, ohne darin durch die Moleküle 6 fixiert zu werden.

Die Trägerpartikel 7 und 8, welche die Filterstruktur 5 passiert haben, gelangen in den Messbereich 9 der sensiblen Oberfläche eines SAW-Sensors 10 und werden vermittels des Magnetfeldes B auf diese Oberfläche gelenkt. Dort angekommen können sie nachgewiesen werden, wobei das Signal des Sensors 10 umso höher ist, je größer die Anzahl der gesättigten Trägerpartikel 7 und 8 ist. Der mit der als Partikellösung ausgebildeten Flüssigkeit gefüllte Behälter und die Filterstruktur 5 werden als eine gemeinsame Kartusche abgebildet, die sich als Wegwerfartikel auswechseln lässt: Nach Abschalten des Magnetfeldes B kann die Oberfläche des Sensors durch Spülen gereinigt werden.

Nach dem in Figur 2 gezeigten Schema ist mit der Eingabe (Pfeil C) kein Analyt in die Flüssigkeit 1 gelangt, weil die von dem Probanden genommene Probe von der nachzuweisenden Substanz frei ist. Durch die Filterstruktur 5 getrieben (Pfeil D), bleiben die Trägerpartikel 11 mit den ungesättigten Rezeptoren 12 darin hängen, da die Rezeptoren 12 an die in der Filterstruktur 5 gebundenen Moleküle 13 koppeln. Es gelangt kein Trägerpartikel 11 in den Raum 9, so dass auch bei Anlegen eines Magnetfeldes B kein Trägerpartikel 11 auf den Sensor 10 trifft.

Während der Messung durchläuft die mit der Probe versehene Partikellösung eine "Messstrecke" innerhalb derer sie mehrere "Analysestationen" passiert. In der ersten Station kommen die Nanopartikel mit der auf die gesuchte Substanz (Analyt) zu untersuchenden Probenflüssigkeit in Kontakt. Ist von der Substanz in der Probe etwas vorhanden, so kann können deren Moleküle an die Rezeptoren binden. Die Rezeptormoleküle werden abgesättigt und können keine weitere Bindung mehr eingehen. Befindet sich hingegen in der Probe keine gesuchte Substanz, bleiben die Rezeptoren aktiv.

In der zweiten Station durchlaufen die Nanopartikel die Matrix der Filterstruktur, die beispielsweise von einem Gewebe gebildet wird und in der die gesuchte Substanz definiert eingebracht und immobilisiert ist. Die Nanopartikel, deren Rezeptoren abgesättigt sind, passieren die Filterstruktur weitgehend ungehindert. Nanopartikel deren Rezeptoren noch "aktiv" sind, da sie noch keine Bindung zum Analyten eingegangen sind, binden hingegen innerhalb der Matrix an die dort immobilisierten Analyte und verbleiben in der Matrix. Die Filterstruktur trennt somit Nanopartikel, die mit dem Analyten aus der Probenflüssigkeit reagiert haben von solchen, bei denen es zu keiner Bindungswechselwirkung gekommen ist. Die dreidimensionale Struktur der Matrix gewährleistet dabei einen sehr hohen Grad an Sensitivität, da so das gesamte Probenvolumen von der Analyse erfasst wird.

Die dritte Station, den Bereich 9, erreichen nur solche Nanopartikel, die den Filter passiert haben. Dabei korreliert die Menge der hier ankommenden Nanopartikel mit der in der Probenflüssigkeit vorhandenen Menge an Analyt.

In diesem letzten Analysebereich können die magnetischen Nanopartikel mit Hilfe eines schaltbaren Elektromagneten auf die Oberfläche des massensensitven Messfühlers gezwungen und hinsichtlich ihrer Menge, respektive ihrer Masse, erfasst werden. Als Messfühler dient dabei ein SAW-Sensor, der gegenüber Änderungen in der Massenbelegung seiner Oberfläche hochsensitiv ist. Die auf der Sensoroberfläche detektierte Menge an Nanopartikeln liefert schließlich eine quantitative Aussage über die Menge an Analyt in der Probenflüssigkeit. In dem beschriebene Verfahren werden magnetische Nanopartikel eingesetzt, um diese gezielt mit dem massensensitiven Messfühler in Kontakt zu bringen.

Die handhabbare Vorrichtung zur Durchführung des Verfahrens weist folgende Komponenten auf:
Eine Messkartusche, umfassend einen mit Flüssigkeit gefüllten Behälter, wobei der Behälter einen Zugang für das Einbringen einer den Analyten aufweisenden Probe aufweist, und umfassend eine Filterstruktur 5,in deren Matrix Moleküle 6 des zu messenden Analyten fest eingebunden sind, wobei sich in der Flüssigkeit für den Analyten spezifische Bindungspartner befinden. Die Messkartusche ist auswechselbar an der Messeinheit angebracht. Zudem ist eine Messeinheit mit einem über die Filterstruktur für die Flüssigkeit zugänglichen Messraum vorhanden, in dem sich ein für den Nachweis der Bindungspartner geeigneter Sensor befindet. Weiterhin weist die Messeinheit Mittel zur Auswertung der vom Sensor generierten Signale und Mittel zur Anzeige der ausgewerteten Ergebnisse und Mittel zum Anlegen eines den Messraum durchdringenden magnetischen Feldes, mit dem die in der Flüssigkeit befindlichen magnetischen Nanopartikel derart beeinflussbar sind, dass sie zum SAW-Sensor hingelenkt werden.

## Patentansprüche

1. Verfahren zum Nachweis eines definierten biorelevanten Analyten, wobei der Analyt in eine mit spezifischen Bindungspartner versehene Flüssigkeit eingebracht wird, wobei Moleküle des Analyten an Rezeptoren von Bindungspartnern selektiv binden, wobei die Flüssigkeit nach Eingabe des Analyten einer Filterstruktur ausgesetzt wird, in der Moleküle desselben Analyten fest gebunden sind, wobei die Filterstruktur Bindungspartner bindet, die freie Rezeptoren aufweisen, wobei Bindungspartner, welche die Filterstruktur passiert haben, mit einem Sensor nachgewiesen werden,
**dadurch gekennzeichnet,**
**dass** als Bindungspartner Trägerpartikel eingesetzt werden, die mit auf den Analyten sensiblen Rezeptoren versehen sind, wobei die Trägerpartikel aus magnetischem Material oder aus elektrisch leitendem und/oder aus polarisierbarem Material sind,
**dass** im Bereich des Sensors angelangte Bindungspartner durch Anlegen eines äußeren Feldes auf einer sensitiven Oberfläche des Sensors gebunden werden, wobei das äußere Feld im Falle magnetischer Trägerpartikel ein Magnetfeld und im Falle elektrisch leitender und/oder polarisierbarer Trägerpartikel ein elektrisches Feld ist.

2. Verfahren nach Anspruch 1,
**dadurch gekennzeichnet,**
**dass** als Trägerpartikel Nanopartikel aus ferromagnetischem Material einer Größe von weniger als 1 Mikrometer insbesondere weniger als 100 Nanometer verwendet werden.

3. Verfahren nach Anspruch 1 oder 2,
**dadurch gekennzeichnet,**
**dass** das äußere Feld vorübergehend für die Dauer der Messung angelegt wird.

4. Verfahren nach einem der vorherigen Ansprüche,
**dadurch gekennzeichnet,**
**dass** nach Abschalten des äußeren Feldes die Oberfläche des Sensors gespült wird.

5. Verfahren nach einem der vorherigen Ansprüche,
**dadurch gekennzeichnet,**
**dass** der Analyt sich in einer Körperflüssigkeit befindet, die an der entsprechenden Körperregion eines Probanden abgenommen wird.

6. Handhabbare Vorrichtung zur Durchführung des Verfahrens nach einem der vorherigen Ansprüche,
**gekennzeichnet durch**
folgende Komponenten:
- Eine Messkartusche, umfassend einen mit Flüssigkeit gefüllten Behälter, wobei der Behälter einen Zugang für das Einbringen einer den Analyten aufweisenden Probe aufweist, und umfassend eine Filterstruktur (5),in deren Matrix Moleküle (6) des zu messenden Analyten fest eingebunden sind, wobei in der Flüssigkeit für den Analyten spezifische Bindungspartner, in Form von Nanopartikeln eingesetzt werden, die mit auf den Analyten sensiblen Rezeptoren versehen sind,
- Eine Messeinheit mit einem über die Filterstruktur (5) für die Flüssigkeit zugänglichen Messraum, in dem sich ein für den Nachweis der Bindungspartner geeigneter SAW-Sensor befindet.
- Zur Messeinheit gehörige Mittel zur Auswertung der vom SAW-Sensor generierten Signale und Mittel zur Anzeige der ausgewerteten Ergebnisse.
- Zur Messeinheit gehörige Mittel zum Anlegen eines den Messraum durchdringenden Feldes, mit dem die Trägerpartikel beeinflussbar sind

7. Vorrichtung nach Anspruch 6,
**dadurch gekennzeichnet,**
**dass** die Messkartusche auswechselbar an der Messeinheit angebracht ist.

8. Vorrichtung nach Anspruch 6 oder 7,
**dadurch gekennzeichnet,**
**dass** die Mittel zum Anlegen eines Feldes ein Magnetfeld generieren.

## Claims

1. Method for the detection of a defined biologically relevant analyte, wherein the analyte is brought into a liquid supplied with a specific binding partner, wherein molecules of the analyte bind selectively at receptors of binding partners, wherein, after input of the analyte, the liquid is exposed to a filtering structure, in which molecules of the same analyte are fixedly bonded, wherein the filtering structure binds binding partners, which comprise free receptors, wherein binding partners, which have passed the filtering structure, are detected by a sensor,
**characterized in,**
**that** carrier particles are used as binding partners, which are provided with receptors sensible for the analyte, wherein the carrier particles are of a magnetic material or of an electric conductive and/or a polarizing material, that binding partners arrived in the area of the sensor are being bonded on a sensitive surface of the sensor by providing an external field, wherein the external field is a magnetic field in case of magnetic carrier particles or an electric field in case of electric conductive and/or polarizing carrier particles.

2. Method according to claim 1,
**characterized in,**
**that** nanoparticles of a ferromagnetic material of a size of less than 1 micrometer especially less than 100 nanometres are used as carrier particles.

3. Method according to claim 1 or 2,
**characterized in,**
**that** the external field is provided temporarily for the duration of the measurement.

4. Method according to any of the preceding claims,
**characterized in,**
**that** the surface of the sensor is flushed after the external field is switched off.

5. Method according to any of the preceding claims,
**characterized in,**
**that** the analyte is located in a body fluid, which is taken from a corresponding body area of a proband.

6. Apparatus capable of being handled for the conduction of the method according to any of the preceding claims,
**characterized by**
following components:
- a measuring cartridge, comprising a container filled with a liquid, wherein the container comprises an access for the insertion of a sample comprising the analyte, and comprising a filtering structure (5), in whose matrix molecules (6) of the analyte to be measured are fixedly embedded, wherein specific binding partners for the analyte in the form of nanoparticles are provided in the fluid, which are provided with receptors sensible for the analyte,
- a measuring unit with a measuring chamber accessible via the filtering structure (5), in which a SAW-sensor is located, which is appropriate for the detection of the binding partner,
- means belonging to the measuring unit for evaluating of the signals generated by the SAW-sensor and means for displaying the evaluated results,
- means belonging to the measuring unit for providing a field penetrating the measuring chamber, with which the carrier particles can be manipulated.

7. Apparatus according to claim 6,
**characterized in,**
**that** the measuring cartridge is attached replaceable at the measuring unit.

8. Apparatus according to claim 6 or 7,
**characterized in,**
**that** the means for providing the filed generates a magnetic field.

## Revendications

1. Procédé de mise en évidence d'un analyte biotique défini, dans lequel l'analyte est incorporé dans un liquide pourvu de partenaires de liaison spécifiques, des molécules de l'analyte se liant sélectivement à des récepteurs de partenaires de liaison, le liquide étant, après incorporation de l'analyte, exposé à une structure de filtration dans laquelle des molécules dudit électrolyte sont liées fixement, la structure de filtration liant les partenaires de liaison qui présentent des récepteurs libres, sachant que les partenaires de liaison qui sont passés dans la structure de filtration sont mis en évidence à l'aide d'un capteur,
**caractérisé en ce**
**qu'**on utilise comme partenaires de liaison des particules porteuses qui sont pourvues de récepteurs sensibles aux analytes, les particules porteuses étant en matériau magnétique ou en matériau conducteur d'électricité et/ou en matériau polarisable,
**que** les partenaires de liaison arrivés au niveau du capteur sont liés par application d'un champ extérieur sur une surface sensitive du capteur, le champ extérieur étant un champ magnétique dans le cas de particules porteuses magnétiques et un champ électrique dans le cas de particules porteuses conductrices d'électricité et/ou polarisables.

2. Procédé selon la revendication 1,
**caractérisé en ce**
**qu'**on utilise comme particules porteuses des nanoparticules en matériau ferromagnétique d'une taille inférieure à 1 micromètre, notamment inférieure à 100 nanomètres.

3. Procédé selon la revendication 1 ou 2,
**caractérisé en ce que**
le champ extérieur est appliqué temporairement pour la durée de la mesure.

4. Procédé selon une des revendications précédentes,
**caractérisé en ce que**
la surface du capteur est lavée après coupure du champ extérieur.

5. Procédé selon une des revendications précédentes,
**caractérisé en ce que**
l'analyte se trouve dans un liquide physiologique qui est prélevé dans la partie corporelle correspondante d'une personne testée.

6. Dispositif maniable pour la réalisation du procédé selon une des revendications précédentes,
**caractérisé par**
les composants suivants :
- une cartouche de mesure, comprenant un récipient rempli de liquide, le récipient présentant un accès pour l'introduction d'un échantillon comportant l'analyte, et comprenant une structure de filtration (5) dans la matrice de laquelle des molécules (6) de l'analyte à mesurer sont liées fixement, sachant que, dans le liquide pour l'analyte, on utilise des partenaires de liaison spécifiques sous forme de nanoparticules qui sont pourvues de récepteurs sensibles aux analytes,
- une unité de mesure avec un espace de mesure accessible via la structure de filtration (5) pour le liquide et dans lequel se trouve un capteur SAW apte à la mise en évidence des partenaires de liaison,
- des moyens faisant partie de l'unité de mesure pour l'exploitation de signaux générés par le capteur SAW et des moyens d'affichage des résultats exploités,
- des moyens faisant partie de l'unité de mesure pour l'application d'un champ traversant l'espace de mesure et permettant d'influer sur les particules porteuses.

7. Dispositif selon la revendication 6,
**caractérisé en ce que**
la cartouche de mesure est installée de manière interchangeable au niveau de l'unité de mesure.

8. Dispositif selon la revendication 6 ou 7,
**caractérisé en ce que**
les moyens d'application d'un champ génèrent un champ magnétique.
